**Europäisches Patentamt**

**European Patent Office**

**Office européen des brevets**

(19)

(11) Veröffentlichungsnummer: **0 045 424**
**B1**

(12) **EUROPÄISCHE PATENTSCHRIFT**

(45) Veröffentlichungstag der Patentschrift:
**19.06.85**

(51) Int. Cl.⁴: **A 01 N 25/02,** A 01 N 53/00,
A 61 K 9/08

(21) Anmeldenummer: **81105673.8**

(22) Anmeldetag: **20.07.81**

(54) Gegen Zecken wirksame Pour-on-Formulierungen.

(30) Priorität: **02.08.80 DE 3029426**

(43) Veröffentlichungstag der Anmeldung:
**10.02.82 Patentblatt 82/6**

(45) Bekanntmachung des Hinweises auf die Patenterteilung:
**19.06.85 Patentblatt 85/25**

(84) Benannte Vertragsstaaten:
**AT BE CH DE FR GB IT LI NL**

(73) Patentinhaber: **BAYER AG, Konzernverwaltung RP
Patentabteilung, D-5090 Leverkusen 1 Bayerwerk (DE)**

(72) Erfinder: **Stendel, Wilhelm, Dr., In den Birken 55,
D-5600 Wuppertal 1 (DE)**
Erfinder: **Voege, Herbert, Dr., Martin-Buberstrasse 41,
D-5090 Leverkusen 3 (DE)**

(56) Entgegenhaltungen:
**EP - A - 0 000 345**
**DE - A - 1 542 752**
**DE - A - 2 614 841**
**GB - A - 2 065 475**
**US - A - 4 020 181**

**Rogoff, W.M. et al., I. Econ. Ent. 53, S. 814-817, 1960**
**Nachr. Chem. Tech. Lab. 26 (1973), Nr. 3, S. 120-122**
**AMATSUO et al., Agr. Biol. Chem. 40 (1) S. 247-249, 1976**

**Die Akte enthält technische Angaben, die nach dem
Eingang der Anmeldung eingereicht wurden und die
nicht in dieser Patentschrift enthalten sind.**

## Beschreibung

Die Erfindung betrifft neue flüssige Aufguß(=Pour-on)-Formulierungen von Wirkstoffen mit Wirkung auf Zecken, die eine bessere Wirksamkeit aufweisen als bisher bekannte Pour-on-Formulierungen für diesen Anwendungszweck.

Eine Pour-on-Formulierung ist dadurch gekennzeichnet, daß der Wirkstoff in einem geeigneten hautverträglichen Lösungsmittel bzw. Lösungsmittelgemisch gegebenenfalls unter Zugabe weiterer Hilfsstoffe gelöst, emulgiert bzw. suspendiert wird und mit Hilfe einer geeigneten Vorrichtung (z. B. mit Hilfe eines Meßbechers, einer Sprühflasche oder einer Dosierspritze) auf die Haut des zu behandelnden Tieres gebracht wird.

In der Veterinärmedizin sind bereits Pour-on-Formulierungen von Insektiziden und Anthelmintika bekanntgeworden (s. dazu Rogoff, W. M. und Kohler, P. H., J. Econ. Ent. 53, 814—817 (1960). Der Ausdruck »Pour-on-Formulierung« oder »Spot-on-Formulierung« ist dem Fachmann geläufig. Eine solche Formulierung stellt eine flüssige Präparation dar, die für die sogenannte »Pour-on-Applikation« geeignet ist und auf die Haut aufgegossen wird (Aufguß-Behandlung).

Beispielsweise werden systemische Phosphorsäureester wie Ruelene, Trichlorphon, Fenthion und andere, die eine stark ausgeprägte insektizide Wirkung besitzen, in Form von Pour-on-Formulierungen zur Bekämpfung von Dassellarven angewendet.

Bisher konnte jedoch eine Zeckenbekämpfung nach dieser Methode nicht mit dem notwendigen Erfolg durchgeführt werden. Solche Pour-on-Formulierungen zeigten zwar eine gewisse, aber bei weitem nicht ausreichende Wirkung. Insbesondere ist es jedoch in Afrika nötig, eine praktisch 100%ige Wirkung auf Zecken zu erreichen, da sonst die von Zecken übertragenen Protozoonosen (z. B. Ostküstenfieber) doch noch auftreten können. Die Zeckenbekämpfung wird daher dort nach wie vor auf herkömmliche Weise durch Tauch(= Dip)-Bäder oder durch Besprühen (Spray) mit wäßrigen Wirkstoff-Emulsionen bzw. Suspensionen durchgeführt.

In der GB 2 065 475 A werden tickizide Pour-on-Formulierungen beschrieben, die einen Träger enthalten, der den Wirkstoff befähigt, durch die Haut eines Tieres zu passieren. Als Träger werden Lösungsmittel wie Alkohole, Öle und Ester eingesetzt, die bevorzugt noch ein Dispergiermittel enthalten können. Trägersysteme, bestehend aus Lösungsmitteln und spreitenden Ölen werden nicht beschrieben.

Aus DE-A-2 614 841 waren bereits Pour-On-Formulierungen bekannt, die gegenüber herkömmlichen Pour-On-Formulierungen aus Rogoff, W. M. und Kohler, P. H., J. Econ. Ent. 53, S. 814—817, 1960, die Resorption des Wirkstoffes durch die Haut verbessern könnten.

Dem ist aber aus DE-A-2 614 841, Seite 7, Absätze 1 und 2 entgegenzusetzen, daß der Zusatz von Spreitölen bei den verwendeten Lösungsmitteln zu keiner größeren benetzten Hautoberfläche führt wie sich im Abklatschtest am Anwendungstier im allgemeinen beweisen läßt. Wenn somit gemäß der Lehre in DE-A-2 614 841 eine verbesserte Wirksamkeit gegen Ektoparasiten, d. h. auf der Haut des Anwendungstieres parasitierende Schädlinge nicht zu erwarten war und eine verbesserte Resorption der Wirkstoffe nicht zum Tragen kommt, weil eine systematische Wirkung der Wirkstoffe ausgeschlossen werden kann (s. unten), so muß die tatsächlich eintretende, meßbare Wirksamkeitsverbesserung der unten beschriebenen Formulierung gegenüber Ektoparasiten, insbesondere gegenüber Zecken, überraschen. Die Verbesserung der Wirksamkeit der Formulierung beruht offensichtlich nicht, wie zunächst angenommen wurde, auf einer verbesserten Benetzung der Hautoberfläche des Behandlungstieres, sondern auf einem bisher ungeklärten Mechanismus, der aber sehr wahrscheinlich mit einer verbesserten Wirkstoffaufnahme durch den Parasiten erklärt werden kann.

Der Hinweis in EP-A-345, demgemäß synthetische Pyrethroide für Pour-On-Formulierungen geeignet sind, legt die Verwendung von Spreitölen in den unten beschriebenen Formulierungen nicht nahe.

Es wurde nun gefunden, daß Pour-On-Formulierungen bestimmter gegen Zecken wirksamer Stoffe, welche 0,1 bis 30 Gewichtsteile eines zeckenwirksamen Stoffes, 10—80 Gewichtsteile eines oder mehrerer spreitender Öle, 20—95 Gewichtsteile eines oder mehrerer hautverträglicher Lösungsmittel und 0 bis 20 Gewichtsteile weiterer Hilfsstoffe enthalten, eine sehr gute Verteilung des gegen Zecken wirksamen Stoffes auf der Tierhaut bewirken.

Eine solche Zeckenbekämpfung im »Pour-on«-Verfahren hat gegenüber den herkömmlichen Methoden folgende Vorteile:

1. Teures Installieren einer Tauch- oder Spray-Anlage entfällt.
2. Keine Schwierigkeiten mit der Wasserversorgung der Dips und Sprays und der Energie-Versorgung der Spray-Anlagen.
3. Herantreiben der Tiere über große Entfernungen entfällt — dadurch kein Gewichtsverlust der Tiere.
4. Keine Schwierigkeiten mit dem Einhalten der optimalen Wirkstoff-Konzentration in den Bädern. (Beim Baden der Tiere entziehen diese der Badeflüssigkeit überproportional Wirkstoff (= Stripping). Dadurch muß in bestimmten Abständen zusätzlich Wirkstoff-Konzentrat dem Bad zugefügt werden, damit der Wirkstoffgehalt im Bad nicht unter die noch wirksame Dosis sinkt).

Die erfindungsgemäßen Pour-on-Formulierungen zeigen eine praktisch 100%ige Wirkung und sind hinsichtlich der Wirkung der Tauch- und Sprühbehandlung vergleichbar. Dies ist um so bemerkenswerter, als das erfindungsgemäße Pour-on-Verfahren auch bei Stoffen anwendbar ist, die nicht systemisch wirken (orale und i. V. Applikation der verwendeten Wirkstoffe zeigten keine tickizide Wirkung). Beispielsweise können im Tauch-und Sprayverfahren zeckenwirksame synthetische Pyrethroide als Pour-on-Formulierungen zur Zeckenbekämpfung angewendet werden.

Dabei ist es überraschend, daß solch geringe Volumen einer solchen Formulierung sich über eine so große Oberfläche verteilen können, wie sie etwa ein Rind besitzt. Dies wird aus den unten aufgeführten Beispielen ersichtlich.

Als für die Herstellung der erfindungsgemäßen Pour-on-Formulierungen einsetzbare Wirkstoffe kommen vor allem in Betracht: Synthetische Pyrethroide, Amidine oder Thioharnstoffe.

Unter spreitenden Ölen werden solche öligen Flüssigkeiten verstanden, die sich auf der Haut besonders gut verteilen. Sie sind als solche in der Kosmetik bekannt. Nach einem Vorschlag von R. Keymer, Pharm. Ind. 32, 577 (1970) können sie z. B. durch ihre Oberflächenspannung gegen Luft charakterisiert werden, die danach weniger als $30 \cdot 10^{-5}$ N/cm betragen sollte. Diese Spreitung kann auch nach dem sogenannten Abklatschtest auf der menschlichen Haut experimentell bestimmt werden (z. B. bei R. Keymer, Pharm. Ind. 32, 577 (1970), oder F. Neuwald, K. E. Fetting und A. Szakall, Fette-Seifen-Anstrichmittel 64, 465 (1962).

Als spreitende Öle kommen praktisch alle Substanzen in Betracht, welche die oben angegebenen Eigenschaften aufweisen. Insbesondere sind die folgenden Verbindungsklassen bzw. Verbindungen geeignet:

Silikonöle verschiedener Viskosität:

Fettsäureester, wie Ethylstearat, Di-n-butyl-adipat, Laurinsäurehexalester, Dipropylen-glykolpelargonat, Ester einer verzweigten Fettsäure mittlerer Kettenlänge mit gesättigten Fettalkoholen $C_{16}-C_{18}$, Isopropylmyristat, Isopropylpalmitat, Capryl/Caprinsäureester von gesättigten Fettalkoholen der Kettenlänge $C_{12}-C_{18}$, Isopropylstearat, Ölsäureoleylester, Ölsäuredecylester, Ethyloleat, Milchsäureethylester, wachsartige Fettsäureester wie künstliche Entenbürzeldrüsenfett, Dibutylphthalat, Adipinsäurediisopropylester, letzterem verwandte Estergemische u. a. Triglyceride, wie Capryl/Caprinsäuretriglycerid, Triglyceridgemische mit Pflanzenfettsäuren der Kettenlänge $C_8-C_{12}$ oder anderen speziell ausgewählten natürlichen Fettsäuren, Partialglyceridgemische gesättigter oder ungesättigter eventuell auch hydroxylgruppenhaltige Fettsäuren, Monodiglyceride der $C_8/C_{10}$-Fettsäuren und andere. Fettalkohole, wie Isotridecylalkohol, 2-Octyldodecanol, Cetylstearylalkohol, Oleylalkohol. Fettsäuren, wie z. B. Ölsäure.

Besonders gut geeignete spreitende Öle sind die folgenden: Isopropylmyristat, Isopropylplamitat, Capryl/Caprinsäureester von gesättigten Fettalkoholen der Kettenlänge $C_{12}-C_{18}$, wachsartige Fettsäureester wie künstliches Entenbürzeldrüsenfett und Silikonöle.

Als für die Herstellung der erfindungsgemäßen Pour-on-Formulierungen geeignete Lösungsmittel kommen im Prinzip alle organischen und anorganischen Lösungsmittel in Frage, die den tickizid wirksamen Stoff in ausreichender Konzentration aufnehmen und das Gewebe nicht schädigen.

Als gut geeignete Lösungsmittel kommen für die Herstellung der erfindungsgemäßen Pour-on-Formulierungen in Betracht:

Alkanole, wie Ethylalkohol, Isopropylalkohole, n-Butylalkohol, Amylalkohol, Octanol. Glykole, wie Propylenglykol, 1,3-Butylenglykol, Ethylglykol, Dipropylenglykolmonomethylether. Aromatische Alkohole wie Benzylalkohol. Carbonsäureester, wie z. B. Ethylacetat, Benzylbenzoat, Butylacetat, Propylencarbonat, Milchsäureethylester. Aliphatische Kohlenwasserstoffe. Öle, die nicht unter die Definition der spreitenden Öle fallen, wie z. B. Baumwollsaatöl, Erdnußöl, Maiskernöl, Olivenöl, Ricinusöl, Sesamöl. Wasser. Ketone, wie z. B. Aceton und Methylethylketon.

Weiterhin sind u. a. Verbindungen wie Dimethylsulfoxid, Dimethylacetamid, Dimethylformamid, N-Methylpyrrolidon, Dioxan, 2-Dimethyl-4-oxymethyl-1,3-dioalan gut als Lösungsmittel geeignet.

Besonders geeignet sind niedere Alkohole mit bis zu 8 Kohlenstoffatomen im Molekül, sowie niedere Ketone wie Methylethylketon und Ether des Ethylenglykols.

Es können bei der Herstellung der erfindungsgemäßen Pour-on-Formulierungen ein oder mehrere Lösungsmittel eingesetzt werden.

Als weitere Hilfsstoffe sind geeignet:

a) Haftvermittler, z. B. Carboxmethylcellulose, Methylcellulose und andere Cellulose- und Stärke-Derivate, Polyacrylate, Alginate, gelatine, Gummiarabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl.

Kolloidale Kieselsäure oder Gemische der aufgeführten Stoffe. Lösungen und Emulsionen können neben dem Wirkstoff die üblichen Trägerstoffe enthalten, wie

b) Tenside (beinhaltet Emulgatoren und Netzmittel), z. B.
   1. anionaktive, wie Na-Laurylsulfat, Fettalkoholethersulfate, Mono/Dialkylpolyglykoletherorthophosphorsäureester-Monoethanolaminsalz,
   2. kationaktive, wie Cetyltrimethylammoniumchlorid,
   3. ampholytische, wie Di-Na-N-lauryl—:—iminodipropionat oder Lecithin,
   4. nicht ionogene, z. B. polyoxethyliertes Ricinzöl, polyoxethyliertes Sorbitan-Monooleat, Sorbitan-Monostearat, Ethylalkohol, Glycerinmonostearat, Polyoxyethylenstearat, Alkylphenolpolyglykolether.

c) Stabilisatoren zur Verhinderung des bei einigen Wirkstoffen eintretenden chemischen Abbaus, wie z. B. Antioxydantien, beispielsweise Tocopherole, Butylhydroxyanisol.

Bevorzugte erfindungsgemäße Pour-On-Formulierungen sind wie folgt zusammengesetzt:

Wirkstoff;

Spreitendes Öl aus einer Reihe Isopropylmyristat, Isopropylpalmitat, Capryl/Caprinsäuretriglycerid, gesättigte Triglyceride natürlicher Fettsäuren, Fettsäureester, die künstlichem Entenbürzeldrüsenfett entsprechen oder Silikonöle;

Lösungsmittel aus der Reihe Isopropanol, Amylalkohol, Methylethylketon, Glykolether, Butylacetat, Milchsäureethylester.

Zur vorliegenden Erfindung gehören auch Aufguß-Zubereitungen in Dosierungseinheiten. Dies bedeutet, daß die Zubereitungen in Form einzelner Teile vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Dosis entspricht.

Als bevorzugte pharmazeutische Zubereitungen seien Lösungen, Suspensionen, Emulsionen und Sprays genannt.

Die erfindungsgemäßen Pour-on-Formulierungen werden hergestellt, indem man den auf Zecken wirksamen Stoff in einem geeigneten hautverträglichen Lösungsmittel bzw. Lösungsmittelgemisch löst, emulgiert bzw. suspendiert, spreitende Öle hinzufügt und gegebenenfalls die weiteren Hilfsstoffe zusetzt.

Die obige Reihenfolge der Verfahrensschritte ist nicht kritisch, sie kann geändert oder die Bestandteile der erfindungsgemäßen Pour-on-Formulierungen können auch gegebenenfalls gleichzeitig unter ständigem Rühren zusammengegeben werden. Bei der Herstellung werden die einzelnen Bestandteile in den vorne angegebenen Mengenverhältnissen zugesetzt.

Die im folgenden aufgeführten erfindungsgemäßen Formulierungen wurden wie oben angegeben hergestellt und vergleichend mit Formulierungen, welche keine spreitenden Öle enthielten, im Hinblick auf die Wirksamkeit getestet.


Beispiel 1


Wirkstoff

| Isopropylmyristat | | 30,00 g | spreitende Öle |
| 2-Octyl-dodecanol | | 20,00 g | |
| Isopropylalkohol | | 28,75 g | |
| | 100 ml = | 83,75 g | |

Die Substanzen werden zusammengewogen und gerührt, bis eine klare Lösung entstanden ist.

4

**0 045 424**

Beispiel 2

Wirkstoff

| | | |
|---|---|---|
| Silikonöl 100 | | 30,00 g |
| Butylacetat | | 59,42 g |
| | 100 ml = | 89,92 g |

**Patentansprüche**

1. Pour-On-Formulierungen von zeckenwirksamen Stoffen, dadurch gekennzeichnet, daß sie

| | |
|---|---|
| 0,1 bis 30 Gewichtsteile | eines tickizid wirksamen Stoffes, |
| 10 bis 80 Gewichtsteile | eines oder mehrerer spreitender Öle, charakterisiert durch eine Oberflächen spannung gegen Luft von $<30 \cdot 10^{-5}$ N/cm, |
| 20 bis 95 Gewichtsteile | eines oder mehrerer hautverträglicher Lösungsmittel und |
| 0 bis 20 Gewichtsteile | weiterer Hilfsstoffe enthalten. |

2. Pour-On-Formulierungen nach Anspruch 1, dadurch gekennzeichnet, daß sie als zeckenwirksamen Stoff ein synthetisches Pyrethroid, ein Amidin oder einen Thioharnstoff enthalten.

3. Verfahren zur Herstellung von Pour-On-Formulierungen zeckenwirksamer Stoffe, dadurch gekennzeichnet, daß

| | |
|---|---|
| 0,1 bis 30 Gewichtsteile | eines tickizid wirksamen Stoffes, |
| 10 bis 80 Gewichtsteile | eines oder mehrerer spreitender Öle, charakterisiert durch eine Oberflächenspannung gegen Luft von $<30 \cdot 10^{-5}$ N/cm, |
| 20 bis 95 Gewichtsteile | eines oder mehrerer hautverträglicher Lösungsmittel und |
| 0 bis 20 Gewichtsteile | weiterer Hilfsstoffe vermischt werden. |

4. Pour-On-Formulierung nach Anspruch 1, dadurch gekennzeichnet, daß sie als spreitendes Öl Isopropylmyristat, Isopropylpalmitat oder Capryl/Caprinsäuretriglycerid enthält.

5. Pour-On-Formulierung nach Anspruch 1, dadurch gekennzeichnet, daß sie als Lösungsmittel Isopropanol, Amylalkohol, Methylethylketon, Glykolether, Butylacetat oder Milchsäureethylester enthält.

6. Pour-On-Formulierung nach Anspruch 1, dadurch gekennzeichnet, daß sie Isopropylmyristat und Isopropanol enthält.

7. Pour-On-Formulierung nach Anspruch 1, dadurch gekennzeichnet, daß sie ein synthetisches Pyrethroid enthält.

8. Pour-On-Formulierung nach Anspruch 1, dadurch gekennzeichnet, daß sie 3-($\beta$-Chlor-$\beta$-p-chlorp-henyl-vinyl)-2,2-dimethylcyclopropancarbonsäurecyano-3-phenoxy-benzylester enthält.

9. Pour-On-Formulierung nach Anspruch 1, dadurch gekennzeichnet, daß sie Isopropylmyristat, Isopropanol und 3-($\beta$-Chlor-$\beta$-p-chlorphenylvinyl)-2,2-dimethylcyclopropancarbonsäurecyano-3-phe-noxy-benzylester enthält.

**Claims**

1. Pour-on-formulations of compounds which are active against ticks, characterised in that they contain

| | |
|---|---|
| 0.1 to 30 parts by weight | of a tickicidally active compound, |
| 10 to 80 parts by weight | of one or more spreading oils, characterised by a surface tension at an air interface of $<30.10^{-5}$ N/cm, |
| 20 to 95 parts by weight | of one or more solvents which are tolerated by skin and |
| 0 to 20 parts by weight | of further auxiliaries. |

2. Pour-on-formulations according to Claim 1, characterised in that they contain a synthetic pyrethroid, an amidine or a thiourea as the compound which is active against ticks.

3. Process for the preparation of pour-onformulations of compounds which are active against ticks, characterised in that

| | |
|---|---|
| 0.1 to 30 parts by weight | of a tickicidally active compound, |
| 10 to 80 parts by weight | of one or more spreading oils, characterised by a surface tension at an air interface of $<30-10^{-5}$ N/cm, |

5

20 to 95 parts by weight of one or more solvents which are tolerated by skin and
0 to 20 parts by weight of further auxiliaries, are mixed.

4. Pour-on formulation according to Claim 1, characterised in that it contains isopropyl myristate, isopropyl palmitate or caprylic/capric acid triglyceride as the spreading oil.

5. Pour-on formulation according to Claim 1, characterised in that contains isopropanol, amyl alcohol, methyl ethyl ketone, glycol ethers, butyl acetate or lactic acid ethyl ester as the solvent(s).

6. Pour-on formulation according to Claim 1, characterised in that it contains isopropyl myristate and isopropanol.

7. Pour-on formulation according to Claim 1, characterised in that it contains a synthetic pyrethroid.

8. Pour-on formulation according to Claim 1, characterised in that it contains 3-($\beta$-chloro-$\beta$-p-chloro-phenylvinyl)-2,2-dimethylcyclopropancarboxylic acid cyano-3-phenoxybenzyl ester.

9. Pour-on formulation according to Claim 1, characterised in that it contains isopropyl myristate, isopropanol and 3-($\beta$-chloro-$\beta$-p-chlorophenylvinyl)-2,2-dimethylcyclopropanecarboxylic acid cyano-3-phenoxybenzyl ester.

## Revendications

1. Formulations »Pour-on« de substances actives contre les tiques, caractérisées en ce qu'elles contiennent:

0,1 à 30 parties en poids d'une substance active tuant les tiques,
10 à 80 parties en poids d'une ou plusieurs huiles d'étalement caractérisées par une tension superficielle contre l'air de $< 30 \cdot 10^{-5}$ N/cm,
20 à 95 parties en poids d'un ou plusieurs solvants compatibles avec la peau, et
0 à 20 parties en poids d'autres substances auxiliaires.

2. Formulations »Pour-on« suivant la revendication 1, caractérisées en ce que, comme substance à activité contre les tiques, elles contiennent un pyréthroïde synthétique, une amidine ou une thiourée.

3. Procédé de préparation de formulations »Pour-on« de substance à activité contre les tiques, caractérisée en ce qu'on mélange:

0,1 à 30 parties en poids d'une substance active tuant les tiques,
10 à 80 parties en poids d'une ou plusieurs huiles d'étalement caractérisées par une tension superficielle contre l'air de $< 30 \cdot 10^{-5}$ N/cm,
20 à 95 parties en poids d'un ou plusieurs solvants compatibles avec la peau, et
0 à 20 parties en poids d'autres substances auxiliaires.

4. Formulation »Pour-on« suivant la revendication 1, caractérisée en ce que, comme huile d'étalement, elle contient le myristate d'isopropyle, le palmitate d'isopropyle ou le triglycéride d'acide caprique/caprylique.

5. Formulation »Pour-on« suivant la revendication 1, caractérisée en ce que, comme solvant, elle contient l'isopropanol, l'alcool amylique, la méthyl-éthyl-cétone, l'éther glycolique, l'acétate de butyle ou l'ester éthylique d'acide lactique.

6. Formulation »Pour-on« suivant la revendication 1, caractérisée en ce qu'elle contient du myristate d'isopropyle et de l'isopropanol.

7. Formulation »Pour-on« suivant la revendication 1, caractérisée en ce qu'elle contient un pyréthroïde synthétique.

8. Formulation »Pour-on« suivant la revendication 1, caractérisée en ce qu'elle contient l'ester cyano-3-phénoxy-benzylique d'acide 3-($\beta$-chloro-$\beta$-p-chlorophényl-vinyl)-2,2-diméthyl-cyclopropane-carboxylique.

9. Formulation »Pour-on« suivant la revendication 1, caractérisée en ce qu'elle contient le myristate d'isopropyle, l'isopropanol et l'ester cyano-3-phénoxy-benzylique d'acide 3-($\beta$-chloro-$\beta$-p-chlorophé-nylvinyl)-2,2-diméthyl-cyclopropane-carboxylique.